# EUROPEAN PATENT APPLICATION

(11) **EP 4 821 522 A1**
(43) Date of publication of application: **14.10.2026**
(21) Application number: 24899765.2
(22) Date of filing: 02.12.2024
(51) Int. Cl.: A61M 5/20

(54) **SOUND PRODUCTION MECHANISM OF PRE-FILLED-AND-SEALED AUTOMATIC INJECTION PEN, AND PRE-FILLED-AND-SEALED AUTOMATIC INJECTION PEN**

(30) Priority: 08.12.2023 CN 202311676225
(71) Applicant: Shanghai Innopac Medical Technology Co., Ltd., Shanghai 201605 (CN)
(72) Inventor: GUO, Rong, Shanghai 201605 (CN); WANG, Yijun, Shanghai 201605 (CN)
(74) Representative: karo IP
(86) International application number: PCT/CN2024/136047
(87) International publication number: WO 2025/119125

(57) **Abstract**

The present disclosure provides a sounding mechanism for a pre-filled auto-injection pen, and a pre-filled auto-injection pen. The sounding mechanism for a pre-filled auto-injection pen comprises: a support frame fixed in a housing of the pre-filled auto-injection pen and configured for initially defining initial positions of a push rod and a sounding member; the sounding member, mounted within the support frame and initially positioned by the support frame, and capable of striking the support frame to generate sound upon completion of injection; the push rod, mounted within the support frame and initially positioned by the support frame, and capable, after being triggered, of driving a pre-filled syringe to move so as to complete automatic needle insertion, and being capable, after reaching a limit position, of driving the sounding member to disengage from the initial position; and a rear cover, fixed at a rear end of the support frame and cooperating with the support frame to initially position the sounding member. By supporting the sounding member through cooperation of the always-stationary support frame and the rear cover, the present disclosure ensures that the sounding member is enabled to be stably supported during the action of the push rod, and is only triggered to generate sound when the push rod reaches the limit position, thereby ensuring the reliability of the injection-completion notification.

## Description

### TECHNICAL FIELD

The present disclosure pertains to the technical field of automatic injection, and specifically relates to a sounding mechanism for a pre-filled auto-injection pen and to a pre-filled auto-injection pen.

### BACKGROUND ART

An auto-injector is a medical device capable of automatically injecting a liquid drug from a pre-filled syringe barrel into a patient's body, and is capable of satisfying the needs of users for self-administration. An auto-injector is a medical device for self-use by patients. Since ordinary patients are generally not professional medical personnel, an auto-injector is typically required to have characteristics such as being easy to use, having high reliability and providing a comfortable user experience, and to be capable of emitting a notification signal upon completion of injection, so as to assist in notifying the user that the injection has been completed.

When existing auto-injectors are in use, puncture and injection are generally achieved by simply pressing a front end of the syringe barrel against a part to be injected, while the user is required to manually hold a rear end of the syringe barrel for movement. When the front end of the auto-injector is positioned at the injection site of the patient, an activation member on the auto-injector is depressed, so as to activate the auto-injector, whereupon the needle is able to rapidly puncture the skin, and the drug is automatically injected via the needle.

CN112076364A discloses an auto-injector which comprises a sounder barrel. The sounder barrel is the principal acting component for emitting a notification signal after the push rod has completed injection. The sounder barrel is sleeved over an outer periphery of the push rod so as to be axially slidable, and a driving elastic member acts on the sounder barrel and drives the sounder barrel to slide toward a rear end of a housing, so that the sounder barrel is able to perform a signal notification function under the elastic force of the driving elastic member. The driving elastic member and the sounder barrel are able to be maintained in an initial assembled state by the restraining action of other components, so as to prevent the sounder barrel from sliding toward the rear end of the housing; the sounder barrel is provided with a pushrod limit fitting structure; a sounder-barrel limit structure is provided between the sounder barrel and an inner tube, for keeping the relative position between the sounder barrel and the inner tube fixed in the initial assembled state. Once the sounder barrel disengages from the axial limit of the inner tube, the sounder barrel moves toward the rear end of the housing under the elastic force of the push rod, and simultaneously emits a notification signal. In this sounding structure, dynamic support is present between the push rod and the sounder barrel, and there exists a situation in which the support is unstable, such that sounding may occur prematurely in a state in which the injection has not yet been completed.

### SUMMARY OF THE INVENTION

In view of the problems set forth in the foregoing description of the background art, an object of the present disclosure is to provide a sounding mechanism for a pre-filled auto-injection pen, and a pre-filled auto-injection pen, having a reliable injection-completion notification function.

The technical solution adopted by the present disclosure is as follows:
A sounding mechanism for a pre-filled auto-injection pen, comprising:
a support frame, the support frame being fixed in a housing of the pre-filled auto-injection pen, the support frame being configured for initially defining initial positions of a push rod and a sounding member;
the sounding member, the sounding member being mounted within the support frame and initially being positioned by the support frame, the sounding member being capable of striking the support frame to generate sound upon completion of injection;
the push rod, the push rod being mounted within the support frame and initially being positioned by the support frame, the push rod being capable, after being triggered, of driving a pre-filled syringe to move so as to complete automatic needle insertion, and being capable, after reaching a limit position, of driving the sounding member to disengage from the initial position; and
a rear cover, the rear cover being fixed at a rear end of the support frame and cooperating with the support frame to initially position the sounding member.

Further, a sounding spring is mounted between a rear end of the sounding member and the rear cover, the sounding spring being configured to drive the sounding member to strike the support frame.

Further, an accommodation cavity for accommodating the sounding spring is provided within the rear cover.

Further, a positioning rod is provided within the support frame, the push rod is sleeved over an outside of the positioning rod, and a trigger spring capable of driving the push rod is provided between the push rod and the positioning rod.

Further, in an initial position of the sounding member, a gap is provided between a rear end of the sounding member and a rear end of the positioning rod, and the rear end of the sounding member is capable of striking the rear end of the positioning rod to generate sound upon completion of injection.

Further, the sounding member comprises a U-shaped sounding body, and both front ends of the U-shaped sounding body are each provided with a cantilever.

Further, a stepped surface for initially limiting the cantilevers is provided at an inside of a front end of the support frame.

Further, a protrusion for pushing the cantilevers out of the stepped surface is provided at a rear end of the push rod.

A pre-filled auto-injection pen, comprising:
a housing having a hollow structure;
a pen cap, the pen cap being snap-fitted on a head portion of the housing and being capable, when removed, of simultaneously detaching an inner protective rubber sleeve of a pre-filled syringe;
a trigger sleeve, the trigger sleeve being movably mounted within the housing, a return spring for resetting being provided axially between the trigger sleeve and the housing, a head portion of the trigger sleeve being exposed outside the housing, the trigger sleeve being capable, when moving from an initial position to a limit position, of actuating a protective sleeve;
the pre-filled syringe, the pre-filled syringe being pre-installed and clamped on a holder, wherein during the movement of the trigger sleeve from the initial position to the limit position, an injection end of the pre-filled syringe is consistently concealed within a needle protective sheath, and wherein, when the injection end of the pre-filled syringe is triggered, the injection end undergoes instantaneous displacement and extends out of the trigger sleeve to achieve automatic needle insertion;
the holder, the holder being fixedly mounted between the trigger sleeve and the pre-filled syringe, and being configured to initially define an initial position of the pre-filled syringe and being capable of providing support during automatic needle insertion of the pre-filled syringe;
the protective sleeve, the protective sleeve being mounted outside the support frame and being initially snap-fitted on the outside of the support frame, the protective sleeve being capable, when driven by the trigger sleeve to move, of triggering the push rod, and being simultaneously capable, after movement, of restricting rearward movement of the trigger sleeve so as to provide needle protection;
wherein the sounding mechanism set forth above is mounted within the housing.

Compared with the prior art, significant advantages of the present disclosure include the following: by supporting the sounding member through cooperation of the always-stationary support frame and the rear cover, the sounding member is enabled to be stably supported during the action of the push rod, and is only triggered to generate sound when the push rod reaches the limit position, thereby ensuring the reliability of the injection-completion notification.

### BRIEF DESCRIPTION OF THE DRAWINGS

FIG. 1 is a sectional structural schematic view of the sounding mechanism of the present disclosure;
FIG. 2 is an operational-flow schematic view of the sounding mechanism of the present disclosure;
FIG. 3 is a sectional structural schematic view of the support frame and the rear cover of the present disclosure after assembly;
FIG. 4 is a structural schematic view of the sounding member of the present disclosure; and
FIG. 5 is an exploded structural schematic view of the pre-filled auto-injection pen of the present disclosure.

Reference signs: 1, support frame; 11, positioning rod; 12, stepped surface; 2, rear cover; 21, accommodation cavity; 3, sounding member; 31, U-shaped sounding body; 32, cantilever; 4, sounding spring; 5, trigger spring; 6, push rod; 61, protrusion; 7, protective sleeve; 8, pre-filled syringe; 9, holder; 10, housing; 20, trigger sleeve; 30, return spring.

### DETAILED DESCRIPTION

The present disclosure is further described below in conjunction with specific embodiments, but the present disclosure is not limited to these specific embodiments. Those skilled in the art shall recognize that the present disclosure encompasses all alternatives, modifications and equivalents that may be included within the scope of the claims.

In the description of the present disclosure, it should be understood that the terms "center", "longitudinal", "lateral", "length", "width", "thickness", "upper", "lower", "front", "rear", "left", "right", "vertical", "horizontal", "top", "bottom", "inner", "clockwise", "counterclockwise" and the like, when used to indicate orientations or positional relationships, are based on the orientations or positional relationships shown in the accompanying drawings, are used merely for convenience in describing the present disclosure and for simplifying the description, and are not intended to indicate or imply that the device or element referred to must have a particular orientation, or be constructed or operated in a particular orientation, and shall therefore not be construed as limiting the present disclosure. Furthermore, the terms "first" and "second" are used for descriptive purposes only, and shall not be construed as indicating or implying relative importance, nor as implicitly indicating the number of technical features indicated. Thus, a feature defined as "first" or "second" may explicitly or implicitly include one or more such features. In the description of the present disclosure, unless otherwise stated, "plural" means two or more, unless otherwise explicitly defined.

In the present disclosure, unless otherwise explicitly specified and defined, the terms "mounted", "linked", "connected", "fixed" and the like shall be understood in a broad sense; for example, they may refer to a fixed connection, a detachable connection or an integral connection; they may refer to a mechanical connection or an electrical connection; they may refer to a direct linkage, or an indirect linkage via an intermediate medium, or to internal communication between two elements. For those of ordinary skill in the art, the specific meanings of the foregoing terms in the present disclosure may be understood according to specific circumstances.

In the present disclosure, unless otherwise explicitly specified and defined, a first feature being "above" or "below" a second feature may include direct contact between the first feature and the second feature, and may also include the first feature and the second feature not being in direct contact but being in contact via a further feature between them. Moreover, the first feature being "over", "above" or "on top of" the second feature includes the first feature being directly above and obliquely above the second feature, or merely indicates that the horizontal level of the first feature is higher than that of the second feature. The first feature being "under", "below" or "beneath" the second feature includes the first feature being directly below and obliquely below the second feature, or merely indicates that the horizontal level of the first feature is lower than that of the second feature.

### Embodiment 1

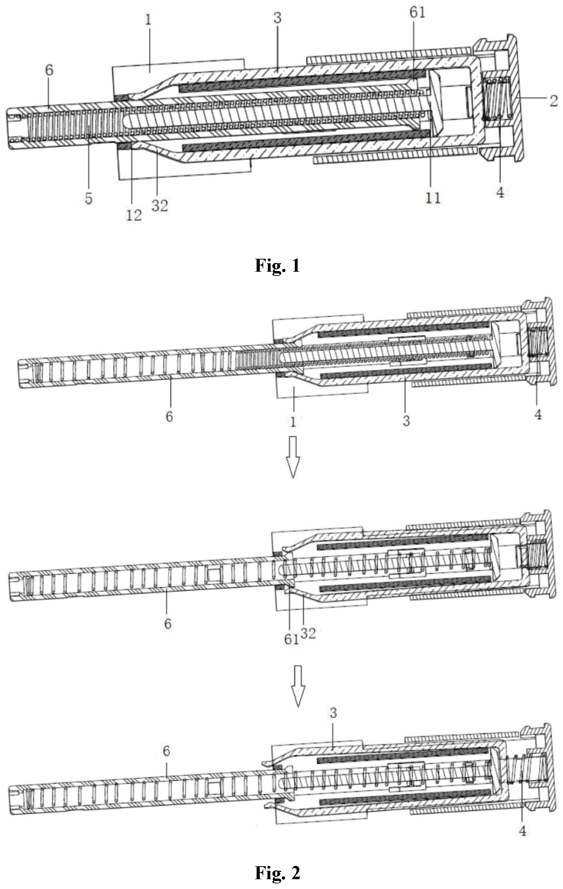

Referring to FIGS. 1 to 4, the present embodiment provides a sounding mechanism for a pre-filled auto-injection pen, comprising:
a support frame 1, the support frame 1 being fixed in an outer housing of the pre-filled auto-injection pen and being configured for initially defining initial positions of a push rod 6 and a sounding member 3;
the sounding member 3, the sounding member 3 being mounted within the support frame 1 and initially being positioned by the support frame 1, the sounding member 3 being capable of striking the support frame 1 to generate sound upon completion of injection;
the push rod 6, the push rod 6 being mounted within the support frame 1 and initially being positioned by the support frame 1, the push rod 6 being capable, after being triggered, of driving a pre-filled syringe to move so as to complete automatic needle insertion, and being capable, after reaching a limit position, of driving the sounding member 3 to disengage from the initial position; and
a rear cover 2, the rear cover 2 being fixed at a rear end of the support frame 1 and cooperating with the support frame 1 to initially position the sounding member 3.

In the present embodiment, a sounding spring 4 is mounted between the rear end of the sounding member 3 and the rear cover 2, the sounding spring 4 being configured to drive the sounding member 3 to strike the support frame 1. An accommodation cavity 21 for accommodating the sounding spring 4 is provided within the rear cover 2.

In the present embodiment, a positioning rod 11 is provided within the support frame 1, the push rod 6 is sleeved over an outside of the positioning rod 11, and a trigger spring 5 capable of driving the push rod 6 is provided between the push rod 6 and the positioning rod 11. In the initial position of the sounding member 3, a gap is provided between the rear end of the sounding member 3 and a rear end of the positioning rod 11, and the rear end of the sounding member 3 is capable of striking the rear end of the positioning rod 11 to generate sound upon completion of injection.

In the present embodiment, the sounding member 3 comprises a U-shaped sounding body 31, and both front ends of the U-shaped sounding body 31 are each provided with a cantilever 32. A stepped surface 12 for initially limiting the cantilevers 32 is provided at an inside of a front end of the support frame 1. A protrusion 61 for pushing the cantilevers 32 out of the stepped surface 12 is provided at a rear end of the push rod 6.

The sounding member 3 of the present disclosure is fixed on the stepped surface 12 of the support frame 1 by means of the cantilevers 32, the rear sounding spring 4 is compressed so as to store energy, and when the push rod 6 has been triggered, the trigger spring 5 inside the push rod is in an energy-storing state, and the push rod 6 is released and triggered. When the push rod 6 reaches the limit position, the protrusion 61 thereof pushes the cantilevers 32 aside, such that the cantilevers 32 slide off the stepped surface 12 of the support frame 1, and strike the rear end of the positioning rod 11 under the elastic force of the sounding spring 4, so as to emit a notification signal sound, the sounding being stable and reliable.

The present disclosure, by supporting the sounding member 3 through cooperation of the always-stationary support frame 1 and the rear cover 2, ensures that the sounding member 3 is enabled to be stably supported during the action of the push rod 6, and is only triggered to generate sound when the push rod 6 reaches the limit position, thereby ensuring the reliability of the injection-completion notification.

### Embodiment 2

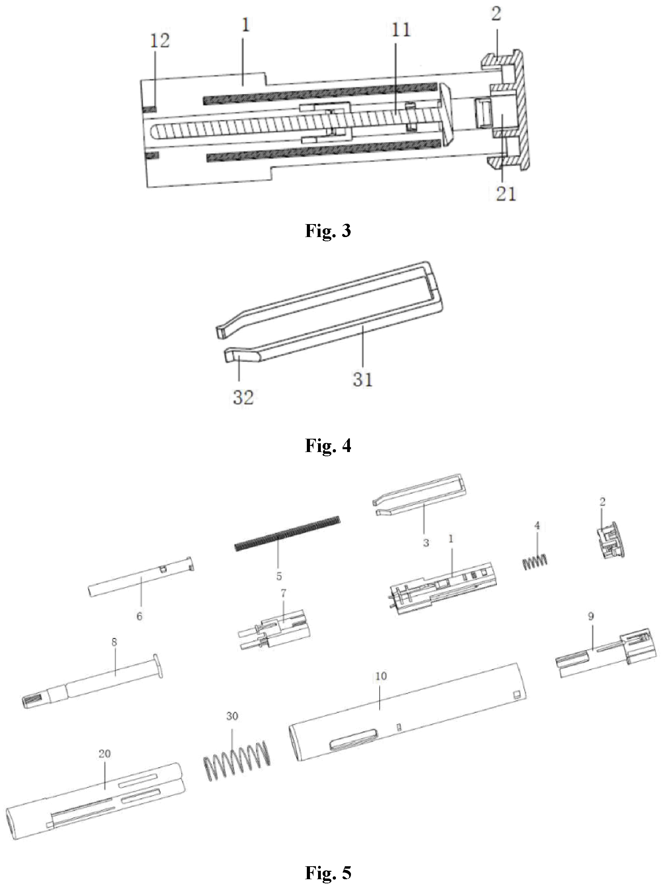

Referring to FIG. 5, the present embodiment provides a pre-filled auto-injection pen, comprising:
a housing 10 having a hollow structure;
a pen cap, the pen cap being snap-fitted on a head portion of the housing and being capable, when removed, of simultaneously detaching an inner protective rubber sleeve of a pre-filled syringe;
a trigger sleeve 20, the trigger sleeve 20 being movably mounted within the housing 10, a return spring 30 for resetting being provided axially between the trigger sleeve 20 and the housing 10, a head portion of the trigger sleeve 20 being exposed outside the housing 10, the trigger sleeve 20 being capable, when moving from an initial position to a limit position, of actuating a protective sleeve 7;
a pre-filled syringe 8, the pre-filled syringe 8 being pre-installed and clamped on a holder 9, wherein during movement of the trigger sleeve 20 from the initial position to the limit position, an injection end of the pre-filled syringe 8 is consistently concealed within a needle protective sheath, and wherein, when the injection end of the pre-filled syringe 8 is triggered, the injection end undergoes instantaneous displacement and extends out of the trigger sleeve 20 to achieve automatic needle insertion;
the holder 9, the holder 9 being fixedly mounted between the trigger sleeve 20 and the pre-filled syringe 8, and being configured to initially define the initial position of the pre-filled syringe 8 and being capable of providing support during automatic needle insertion of the pre-filled syringe 8; and
the protective sleeve 7, the protective sleeve 7 being mounted outside the support frame 1 and being initially snap-fitted on the outside of the support frame 1, the protective sleeve 7 being capable, when driven by the trigger sleeve 20 to move, of triggering the push rod 6, and being simultaneously capable, after movement, of restricting rearward movement of the trigger sleeve 20 so as to provide needle protection;
wherein the sounding mechanism according to Embodiment 1 is mounted within the housing 10.

In the present embodiment, the pen cap is snap-fitted on the head portion of the housing 10 and is capable, when removed, of simultaneously detaching the inner protective rubber sleeve of the pre-filled syringe 8. Specifically, the pen cap is provided with pen-cap snap catches arranged symmetrically, the pen-cap snap catches being initially snap-engaged with the inner protective rubber sleeve of the pre-filled syringe. The inner protective rubber sleeve of the pre-filled syringe 8, upon the removal of the pen cap, disengages from the pre-filled syringe 8 to expose a needle.

In the present embodiment, the housing 10 has a hollow structure; the housing 10 is provided with a viewing window for checking the condition of the liquid drug, for checking whether the position of the pre-filled syringe is correct, and for checking the infusion condition after injection completion.

In the present embodiment, the trigger sleeve 20 is movably mounted within the housing 10, and the return spring 30 for resetting is provided axially between the trigger sleeve 20 and the housing 10; the head portion of the trigger sleeve 20 is exposed outside the housing 10, the trigger sleeve 20 being capable, when moving from the initial position to the limit position, of actuating a trigger assembly. Specifically, the trigger sleeve 20 comprises a trigger sleeve body for protecting the needle; symmetrically arranged trigger portions extend from a tail end of the trigger sleeve body, each trigger portion being provided with a slotted hole for accommodating movement of a second cantilever of the holder, and each trigger portion being provided, on an inner side thereof at one end of the slotted hole, with a radially protruding protective catch for delimiting the initial position of the second cantilever of the holder 9. After movement, a rear end of the trigger portion contacts an interlock portion of the protective sleeve 7, whereby the protective sleeve 7 is driven to move rearward, so as to realize the triggering of the trigger assembly.

In the present embodiment, the pre-filled syringe 8 is pre-installed and clamped on the holder 9; during movement of the trigger sleeve 20 from the initial position to the limit position, the injection end of the pre-filled syringe 8 is consistently concealed within the needle protective sheath, and when the injection end of the pre-filled syringe 8 is triggered, the injection end undergoes instantaneous displacement and extends out of the trigger sleeve 20 to achieve automatic needle insertion. Specifically, the pre-filled syringe 8, when pre-installed, is clamped at the position of the second cantilever on the holder 9, this position being the initial position of the pre-filled syringe 8. Upon triggering, the trigger sleeve 20 moves from the initial position to the limit position; when the trigger sleeve 20 is at the limit position, the injection end of the pre-filled syringe 8 is concealed within the trigger sleeve 20, and when the injection end of the pre-filled syringe 8 is triggered, the injection end undergoes instantaneous displacement, moving from the initial position to a stepped surface on the holder 9 and extending beyond the trigger sleeve 20, so as to achieve automatic needle insertion. The injection end is of a needle structure, and is initially provided with the inner protective rubber sleeve for protecting the injection end. The pre-filled syringe 8 is pre-filled with a liquid and sealed by a rubber stopper. A protruding tail end of the pre-filled syringe 8 is arranged protrusively and is capable of being delimited at the initial position by the holder 9, and cooperates with the stepped surface on the holder 9 to limit the distance of movement.

In the present embodiment, the holder 9 is fixedly mounted between the trigger sleeve 20 and the pre-filled syringe 8, and is configured to initially define the initial position of the pre-filled syringe 8 and to be capable of providing support during automatic needle insertion of the pre-filled syringe 8. The holder 9 is symmetrically provided with second cantilevers, the second cantilevers being arranged in correspondence with the slotted holes of the trigger sleeve 20; an outer side of an end portion of each second cantilever is provided with an outer catch which initially cooperates with the protective catch of the trigger sleeve 20 to delimit the position of the second cantilever; an inner side of the end portion of each second cantilever is provided with an inner catch which initially cooperates with the protruding tail end of the pre-filled syringe 8 so as to support the pre-filled syringe 8. An inner wall of the holder 9 is provided with the stepped surface for limiting movement of the pre-filled syringe 8; the inner wall of the holder 9 is provided, from the stepped surface to the tail end thereof, with upstanding rib portions arranged axially for providing support during automatic needle insertion of the pre-filled syringe 8. After the outer catches of the second cantilevers of the holder 9 disengage from the protective catches, the second cantilevers are capable of being deformed within the slotted holes, such that the pre-filled syringe 8, under the action of the push rod 6, breaks through the inner catches and moves down to the stepped surface. A silicone washer may be provided on the stepped surface, for protecting the protruding tail end of the pre-filled syringe 8, so as to prevent the protruding tail end from rupturing during movement thereof.

In the present embodiment, the support frame 1 is fixed within the housing 10, and is configured to initially define the initial positions of the push rod 6, the sounding member 3 and the protective sleeve 7. Specifically, the positioning rod 11 is provided within the support frame 1, the push rod 6 is sleeved over the outside of the positioning rod 11, and the trigger spring 5 capable of driving the push rod 6 is provided between the push rod 6 and the positioning rod 11. In the initial position of the sounding member 3, a gap is provided between the rear end of the sounding member 3 and the rear end of the positioning rod 11, and the rear end of the sounding member 3 is capable of striking the rear end of the positioning rod 11 to generate sound upon completion of injection. The support frame 1 is provided with a first positioning groove for initially cooperating with the protective sleeve 7 for positioning; the support frame 1 is provided, on a rear side of the first positioning groove, with a stop portion for cooperating with the protective sleeve 7 after movement for positioning; the support frame 1 is provided with a first cantilever, and an inner wall of the first cantilever is provided with a first positioning catch for initially cooperating with the push rod 6 for positioning. The first cantilever is initially press-fitted by the protective sleeve 7, whereby the first positioning catch is snap-engaged on the push rod 6. The rear end of the support frame 1 is provided with a second positioning groove for fixing the rear cover 2. An outer surface of the support frame 1 is symmetrically provided with arc-shaped projection portions for spreading the protective sleeve 7 to abut against the trigger sleeve 20.

In the present embodiment, the push rod 6 is mounted within the support frame 1 and is initially positioned by the support frame 1, and, after the protective sleeve 7 has been triggered, drives, under the impetus of the trigger spring 5, the pre-filled syringe 8 to move so as to complete automatic needle insertion. Specifically, the push rod 6 is provided with a third positioning groove for cooperating with the first positioning catch for initial positioning; a head portion of the push rod 6 is a pushing end which contacts the rubber stopper of the pre-filled syringe 8; an interior of the push rod 6 is a hollow structure for accommodating the trigger spring 5.

In the present embodiment, the protective sleeve 7 is mounted outside the support frame 1, is initially snap-fitted on the outside of the support frame 1, and is capable, when driven by the trigger sleeve 20 to move, of triggering the push rod 6, and is simultaneously capable, after movement, of restricting rearward movement of the trigger sleeve 20 to provide needle protection. Specifically, a front-end wall surface of the protective sleeve 7 is provided with a notch, so as to form a deformable structure at the front end. The deformable front ends are arranged symmetrically; the deformable front ends are capable of being spread under the action of the support frame 1 so as to abut against the rear end of the trigger sleeve 20. The interlock portion is provided protrusively on an outer side surface of each deformable front end; the interlock portion is driven by the rear end of the trigger sleeve 20 so as to drive the protective sleeve 7 to move rearward. An inner side of the rear end of the protective sleeve 7 is provided with a second positioning catch; the second positioning catch is capable of being snap-engaged on the support frame 1 both initially and after movement. The protective sleeve 7 is provided with a first sliding groove for accommodating the projection portion initially and for providing a space for the spreading of the first cantilever after movement. By virtue of the arrangement of the first sliding groove, the protective sleeve 7 does not undergo deformation initially or during the forepart of the rearward movement process; only when the deformable front end moves to the projection portion is the deformable front end spread and deformed, such that the deformable front end is enabled to restrict rearward movement of the trigger sleeve 20. Moreover, when the first sliding groove moves to the position of the first cantilever, the first cantilever is enabled to be spread by the push rod 6, such that the push rod 6 leaves the initial position and moves under the action of the trigger spring 5.

In the present embodiment, the sounding member 3 comprises the U-shaped sounding body 31, both front ends of the U-shaped sounding body 31 being each provided with a cantilever 32. The stepped surface 12 for initially limiting the cantilevers 32 is provided at an inside of the front end of the support frame 1. The protrusion 61 for pushing the cantilevers 32 out of the stepped surface 12 is provided at the rear end of the push rod 6.

In the present embodiment, the rear cover 2 is fixed at the rear end of the support frame 1 and cooperates with the support frame 1 to initially position the sounding member 3.

The sounding member 3 of the present disclosure is fixed on the stepped surface 12 of the support frame 1 by means of the cantilevers 32, the rear sounding spring 4 is compressed so as to store energy, and when the push rod 6 has been triggered, the trigger spring 5 inside the push rod is in an energy-storing state, and the push rod 6 is released and triggered. When the push rod 6 reaches the limit position, the protrusion 61 thereof pushes the cantilevers 32 aside, such that the cantilevers 32 slide off the stepped surface 12 of the support frame 1, and strike the rear end of the positioning rod 11 under the elastic force of the sounding spring 4, so as to emit a notification signal sound, the sounding being stable and reliable.

The present disclosure, by supporting the sounding member 3 through cooperation of the always-stationary support frame 1 and the rear cover 2, ensures that the sounding member 3 is enabled to be stably supported during the action of the push rod 6, and is only triggered to generate sound when the push rod 6 reaches the limit position, thereby ensuring the reliability of the injection-completion notification.

## Claims

1. A sounding mechanism for a pre-filled auto-injection pen, **characterized in that** the sounding mechanism comprises:
a support frame (1), the support frame (1) being fixed in a housing (10) of the pre-filled auto-injection pen, the support frame (1) being configured for initially defining initial positions of a push rod (6) and a sounding member (3);
the sounding member (3), the sounding member (3) being mounted within the support frame (1) and initially being positioned by the support frame (1), the sounding member (3) being capable of striking the support frame (1) to generate sound upon completion of injection;
the push rod (6), the push rod (6) being mounted within the support frame (1) and initially being positioned by the support frame (1), the push rod (6) being capable, after being triggered, of driving a pre-filled syringe (8) to move so as to complete automatic needle insertion, and being capable, after reaching a limit position, of driving the sounding member (3) to disengage from the initial position; and
a rear cover (2), the rear cover (2) being fixed at a rear end of the support frame (1) and cooperating with the support frame (1) to initially position the sounding member (3).

2. The sounding mechanism for a pre-filled auto-injection pen according to claim 1, **characterized in that** a sounding spring (4) for driving the sounding member (3) to strike the support frame (1) is mounted between a rear end of the sounding member (3) and the rear cover (2).

3. The sounding mechanism for a pre-filled auto-injection pen according to claim 2, **characterized in that** an accommodation cavity (21) for accommodating the sounding spring (4) is provided within the rear cover (2).

4. The sounding mechanism for a pre-filled auto-injection pen according to claim 1, **characterized in that** a positioning rod (11) is provided within the support frame (1), the push rod (6) is sleeved over an outside of the positioning rod (11), and a trigger spring (5) capable of driving the push rod (6) is provided between the push rod (6) and the positioning rod (11).

5. The sounding mechanism for a pre-filled auto-injection pen according to claim 4, **characterized in that**, in the initial position of the sounding member (3), a gap is provided between a rear end of the sounding member (3) and a rear end of the positioning rod (11), and the rear end of the sounding member (3) is capable of striking the rear end of the positioning rod (11) to generate sound upon completion of injection.

6. The sounding mechanism for a pre-filled auto-injection pen according to claim 1, **characterized in that** the sounding member (3) comprises a U-shaped sounding body (31), both front ends of the U-shaped sounding body (31) being each provided with a cantilever (32).

7. The sounding mechanism for a pre-filled auto-injection pen according to claim 6, **characterized in that** a stepped surface (12) for initially limiting the cantilevers (32) is provided at an inside of a front end of the support frame (1).

8. The sounding mechanism for a pre-filled auto-injection pen according to claim 7, **characterized in that** a protrusion (61) for pushing the cantilevers (32) out of the stepped surface is provided at a rear end of the push rod (6).

9. A pre-filled auto-injection pen, comprising:
a housing (10) having a hollow structure;
a pen cap, the pen cap being snap-fitted on a head portion of the housing (10) and being capable, when removed, of simultaneously detaching an inner protective rubber sleeve of a pre-filled syringe (8);
a protective sleeve (7);
a trigger sleeve (20), the trigger sleeve (20) being movably mounted within the housing (10), a return spring (30) for resetting being provided axially between the trigger sleeve (20) and the housing (10), a head portion of the trigger sleeve (20) being exposed outside the housing, the trigger sleeve (20) being capable, when moving from an initial position to a limit position, of actuating the protective sleeve (7);
the pre-filled syringe (8), the pre-filled syringe (8) being pre-installed and clamped on a holder (9), wherein during the movement of the trigger sleeve (20) from the initial position to the limit position, an injection end of the pre-filled syringe (8) is consistently concealed within a needle protective sheath, and wherein, when the injection end of the pre-filled syringe (8) is triggered, the injection end undergoes instantaneous displacement and extends out of the trigger sleeve (20) to achieve automatic needle insertion;
the holder (9), the holder (9) being fixedly mounted between the trigger sleeve (20) and the pre-filled syringe (8), and being configured to initially define an initial position of the pre-filled syringe (8) and being capable of providing support during automatic needle insertion of the pre-filled syringe (8); and
the protective sleeve (7) being mounted outside a support frame (1) and being initially snap-fitted on the outside of the support frame (1), the protective sleeve (7) being capable, when driven by the trigger sleeve (20) to move, of triggering a push rod (6), and being simultaneously capable, after movement, of restricting rearward movement of the trigger sleeve (20) so as to provide needle protection;
**characterized in that** the sounding mechanism according to any one of claims 1 to 8 is mounted within the housing (10).
